# FASCICULE DE BREVET EUROPEEN

(11) **EP 3 232 966 B1**
(45) Date de publication et mention de la délivrance du brevet: **31.10.2018**
(21) Numéro de dépôt: 15832815.3
(22) Date de dépôt: 21.12.2015
(51) Int. Cl.: A61B 17/88

(54) **DISPOSITIF EN KIT POUR LE MELANGE ET L'INJECTION D'UN CIMENT OSSEUX**
VORRICHTUNG IN FORM EINES KITS ZUM MISCHEN UND INJIZIEREN EINES KNOCHENZEMENT
DEVICE IN THE FORM OF A KIT FOR MIXING AND INJECTING A BONE CEMENT

(30) Priorité: 19.12.2014 FR 1462974
(43) Date de publication de la demande: 25.10.2017
(73) Titulaire: Teknimed, 65500 Vic-en-Bigorre (FR)
(72) Inventeur: LEONARD, Alain, 207 Nosy Be (MG); LEONARD, Carole, 31380 Paulhac (FR); HALBIN, Gautier, 31000 Toulouse (FR); SENDER, Cyril, 31500 Toulouse (FR)
(74) Mandataire: Cabinet Morelle & Bardou SC
(86) Numéro de dépôt international: PCT/FR2015/053697
(87) Numéro de publication internationale: WO 2016/097665

(56) Documents cités:
- EP-A1- 1 614 403
- WO-A2-2005/048886
- FR-A1- 2 967 344
- US-A1- 2011 114 212

## Description

### Domaine Technique

La présente invention se rapporte de manière générale aux dispositifs utilisés dans la chirurgie osseuse, et plus particulièrement à un dispositif en kit pour le mélange et l'injection d'un ciment osseux.

### Arrière-plan Technologique

L'injection de ciment osseux est réalisée dans diverses interventions chirurgicales, comme les interventions de vertébroplastie, afin de consolider la structure de l'os après traumatisme, usure naturelle ou dégénérescence due à une maladie dégénérative.

Les ciments osseux actuellement utilisés ont la particularité d'être composés d'au moins deux substances, une poudre et un liquide, qui ont besoin d'être mélangées afin d'obtenir une composition homogène. Afin de solidifier et renforcer, par exemple, une vertèbre fragilisée, le ciment ainsi formé doit ensuite être inséré par injection dans le corps vertébral de la vertèbre à traiter.

### Art Antérieur

On connaît des dispositifs permettant à la fois la réalisation du mélange des composants du ciment osseux et, ensuite, l'injection transcutanée du ciment osseux ainsi obtenu au niveau du site cible dans le corps du patient.

Par exemple, le document WO 2005/048886 divulgue divers modes de réalisation d'un appareil de mélange et d'injection de ciment osseux. L'appareil comprend un premier corps cylindrique avec une chambre de mélange, ainsi qu'un second corps cylindrique avec une chambre de décharge. Le ciment est réalisé dans la chambre de mélange. La chambre de décharge peut ensuite être mise en communication fluidique avec la chambre de mélange, pour admettre le ciment osseux. Le ciment osseux est alors injecté dans le corps du patient depuis la chambre de décharge.

Toutefois, les appareils proposés sont de réalisation complexe. Leur utilisation peut de ce fait se révéler hasardeuse, alors qu'une intervention chirurgicale ne peut souffrir aucune forme d'aléa quant au matériel chirurgical utilisé et à la disponibilité du ciment osseux au moment précis où le praticien en aura besoin pour réaliser l'injection.

### Résumé de l'Invention

L'invention vise à supprimer, ou du moins atténuer, tout ou partie des inconvénients de l'art antérieur précités, en proposant un dispositif pour le mélange et l'injection d'un ciment osseux qui soit plus simple d'utilisation.

A cet effet, un premier aspect de l'invention propose un dispositif en kit pour le mélange et l'injection d'un ciment osseux, comprenant un module principal, d'une part, et des accessoires comprenant un capuchon de fermeture amovible et une pluralité de modules fonctionnels, d'autre part, lesdits accessoires étant adaptés pour être chacun connecté ou non au module principal en fonction de phases respectives d'utilisation du dispositif, dans lequel :
- le module principal est un corps cylindrique creux ayant une extrémité proximale et une extrémité distale ouvertes, adaptées pour être connectées chacune à un ou plusieurs des accessoires du dispositif, selon les phases d'utilisation ;
- un des modules fonctionnels est un ensemble de mélange, adapté pour être connecté, dans une phase de mélange, à l'extrémité proximale du module principal alors que l'extrémité distale dudit module principal est fermée par le capuchon de fermeture, et pour permettre à un utilisateur de réaliser le mélange d'au moins deux composés du ciment osseux dans le corps du module principal ;
- un autre des modules fonctionnels est un ensemble d'injection, adapté pour être connecté, dans une phase de transfert venant ensuite de la phase de mélange ainsi que dans une phase d'injection venant ensuite de ladite phase de transfert, à l'extrémité distale du module principal à la place du capuchon de fermeture, et pour permettre à l'utilisateur de réaliser l'injection du ciment osseux ;
- un autre encore des modules fonctionnels, distinct de l'ensemble de mélange et sans aucun élément commun avec l'ensemble de mélange, est un ensemble de transfert, adapté pour être connecté, dans la phase de transfert, à l'extrémité proximale du module principal à la place de l'ensemble de mélange et pour permettre à l'utilisateur de transférer du ciment osseux depuis le corps du module principal vers l'ensemble d'injection.

La simplicité du dispositif proposé par rapport aux appareils de l'art antérieur réside dans le fait qu'il s'agit d'un dispositif en kit d'un type particulièrement abouti, dans lequel l'ensemble de mélange et l'ensemble de transfert sont deux ensembles distincts et indépendants l'un de l'autre, sans aucun élément en commun, et adaptés pour se connecter tour à tour, c'est-à-dire successivement, à la même extrémité du corps cylindrique du module principal ou corps de mélange. Toutefois, le corps de mélange du module principal assure la transition, en ce sens que le mélange qui y est réalisé par l'ensemble de mélange en est ensuite expulsé par l'ensemble de transfert, sans qu'une quelconque intervention de l'utilisateur ne soit nécessaire en dehors de la déconnexion de l'ensemble de mélange et de la connexion de l'ensemble de transfert en lieu et place dudit ensemble de mélange.

Dans un second aspect, l'invention concerne également un procédé d'utilisation du kit selon le premier aspect, plus particulièrement d'un procédé de mélange et d'injection d'un ciment osseux à l'aide d'un dispositif en kit comprenant un module principal ayant la forme d'un corps cylindrique creux avec une extrémité proximale et une extrémité distale ouvertes d'une part, et des accessoires comprenant un capuchon de fermeture amovible et une pluralité de modules fonctionnels, d'autre part, le procédé comprenant les étapes consistant à :
a) dans une phase de mélange, connecter un des modules fonctionnels ou ensemble de mélange à l'extrémité proximale du module principal alors que l'extrémité distale dudit module principal est fermée par le capuchon de fermeture, et réaliser le mélange d'au moins deux composés du ciment osseux dans le corps du module principal ;
b) dans une phase de transfert venant ensuite de la phase de mélange :
   b1) connecter un autre des modules fonctionnels ou ensemble de transfert, distinct de l'ensemble de mélange et sans aucun élément commun avec l'ensemble de mélange, à l'extrémité proximale du module principal à la place de l'ensemble de mélange, et
   b2) connecter un autre encore des modules fonctionnels ou ensemble d'injection à l'extrémité distale du module principal à la place du capuchon de fermeture,
   b3) transférer du ciment osseux depuis le corps du module principal vers l'ensemble d'injection ; et,
c) dans une phase d'injection venant ensuite de la phase de transfert, réaliser l'injection du ciment osseux.

Aucun des éléments de l'ensemble de mélange participant à la fonction de mélange ne risque d'interférer avec un élément quelconque de l'ensemble de transfert. Réciproquement, aucun des éléments de l'ensemble de transfert participant à la fonction de transfert du ciment dans l'ensemble d'injection (ou seringue) ne risque d'interférer avec un élément quelconque de l'ensemble de mélange. On diminue ainsi le risque d'un dysfonctionnement ou d'une erreur de manipulation pouvant conduire à l'échec des opérations de réalisation du mélange et de transfert du ciment ainsi réalisé dans la seringue.

### Brève Description des Dessins

D'autres caractéristiques et avantages de l'invention apparaîtront encore à la lecture de la description qui va suivre. Celle-ci est purement illustrative et doit être lue en regard des dessins annexés sur lesquels :
- la Figure 1, est une vue en trois dimensions d'un mode de réalisation du module principal du dispositif avec deux de ses accessoires ;
- la Figure 2 est une en coupe du module de la Figure 1 avec les deux accessoires installés ;
- La Figure 3 est une vue en coupe de l'ensemble de mélange de la Figure 4 ;
- La Figure 4 est une vue de côté d'un mode de réalisation de l'ensemble de mélange ;
- La Figure 5 est une vue en coupe de l'ensemble de mélange de la Figure 4 connecté au module principal de la Figure 1 ;
- La Figure 6 est une vue en coupe de l'ensemble de transfert de la Figure 7 ;
- La Figure 7 est une vue de côté d'un mode de réalisation de l'ensemble de transfert ;
- La Figure 8 est une vue en coupe de l'ensemble de transfert de la Figure 7 connecté au module principal de la Figure 1 ;
- La Figure 9 une vue de côté de l'ensemble de transfert de la Figure 7 connecté au module principal de la Figure 1 ;
- La Figure 10 est une vue en coupe de l'ensemble de transfert de la Figure 7 connecté au module principal de la Figure 1, après retournement haut/bas ;
- La Figure 11 est une vue en trois dimensions de l'ensemble de transfert de la Figure 7 connecté au module principal de la Figure 1, et d'un mode de réalisation de l'ensemble d'injection ;
- La Figure 12 est une vue de côté des mêmes éléments qu'à la Figure 11 ;
- La Figure 13 est une vue en coupe des éléments de la Figure 12 ;
- La Figure 14 est une vue de côté de l'ensemble de transfert de la Figure 7 connecté au module principal de la Figure 1, et de l'ensemble d'injection de la Figure 11 également connecté audit module principal ;
- La Figure 15 est une vue en coupe correspondant à la vue de la Figure 14, avant le début de la phase de transfert ;

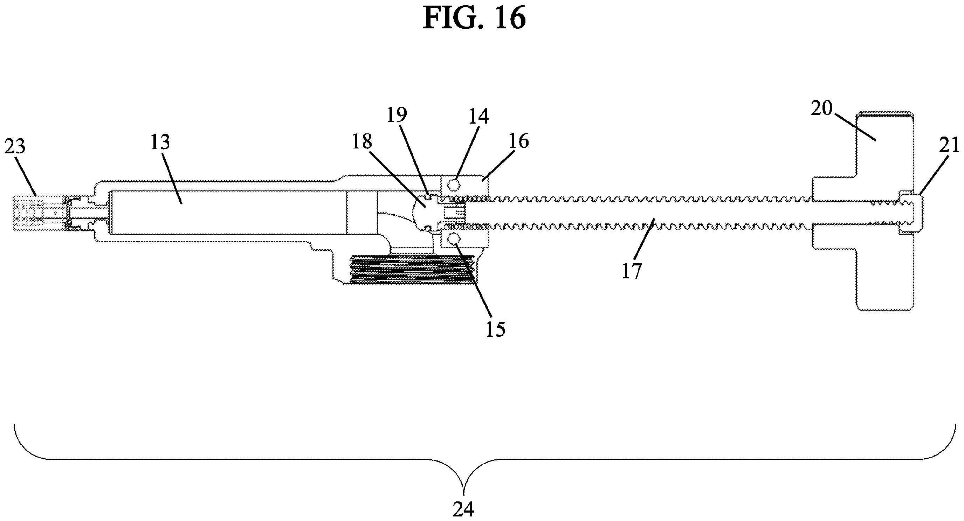

- La Figure 16 est une vue en coupe de l'ensemble d'injection de la Figure 11 ;

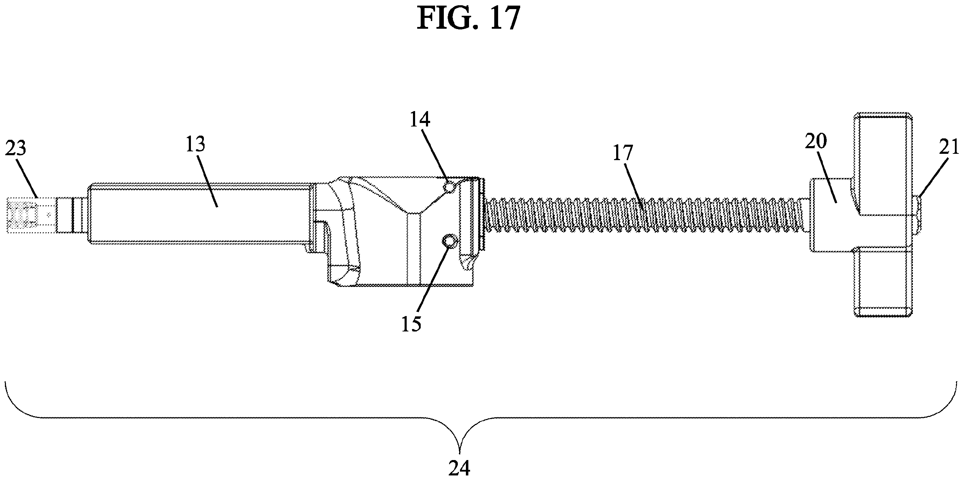

- La Figure 17 est une vue de côté de l'ensemble d'injection de la Figure 11 ;
- La Figure 18 est une vue en coupe du piston de l'ensemble d'injection de la Figure 11 ;
- La Figure 19 est une vue en coupe d'un détail de la tête de piston du piston de l'ensemble d'injection de la Figure 11 ;
- La Figure 20 est une vue en coupe du corps allongé de l'ensemble d'injection de la Figure 11 ;
- La Figure 21 est une vue en coupe montrant les mêmes éléments que la Figure 15, après la phase de transfert mais avant la phase d'injection ; et,
- La Figure 22 est une vue en coupe montrant les mêmes éléments que la Figure 15, dans une configuration du kit lorsqu'il est prêt pour l'injection.

### Description détaillée de modes de réalisation

Dans les dessins et dans la description qui va suivre, les mêmes éléments sont désignés par des mêmes références aux figures des dessins.

Il va être décrit des modes de réalisation d'un dispositif en kit pour le mélange et l'injection d'un ciment osseux, et d'un procédé d'utilisation dudit kit.

Un tel kit peut être utilisé en milieu chirurgical pour la réparation des traumatismes des os et des articulations, par exemple pour le comblement du tissu osseux spongieux ou pour favoriser la fixation d'implants artificiels au squelette d'un patient.

Dans de telles applications de chirurgie osseuse (cimentoplastie), le kit permet à la fois la préparation et l'injection, au niveau d'un site cible, d'un ciment fluide à usage médical ayant des propriétés mécaniques, chimiques et de biocompatibilité adaptées, par exemple du ciment acrylique.

Une intervention chirurgicale de vertébroplastie percutanée, par exemple, consiste à injecter sous contrôle radioscopique ou par scanner, un ciment au sein d'une vertèbre fragilisée, fracturée ou présentant toute autre lésion osseuse, pour la consolider. D'autres applications du kit comprennent la cyphoplastie, qui consiste à injecter du ciment dans le corps vertébral préalablement traité par des ballonnets gonflables, dans le but de redonner une partie de leur volume aux vertèbres tassées.

De tels ciments osseux sont des polymères organiques de composition variable, par exemple à base d'un polymère de méthacrylate. Les polymères organiques peuvent être réalisés à partir d'un mélange binaire, par exemple d'un pré-polymère, généralement du PMMA (poly-(méthacrylate de méthyle)), et d'un monomère, généralement du MMA (méthacrylate de méthyle), réagissant en présence d'un activateur de polymérisation ainsi éventuellement que d'autres adjuvants, selon les applications envisagées.

La plupart des ciments disponibles sont présentés sous forme de deux composants séparés. D'autre part une poudre comprenant principalement des billes de pré-polymère. Et d'autre part un liquide contenant principalement le monomère. On incorpore en général l'initiateur, par exemple du peroxyde de benzoyle (BPO), à la poudre, tandis que le liquide contient un activateur chimique (catalyseur) tel que la di-méthyl-para-toluidine (DMPT). La réaction de polymérisation démarre lorsque les deux composants sont mélangés.

Le kit pour le mélange et l'injection d'un ciment osseux, comprend un module principal, d'une part, et des accessoires, d'autre part, lesdits accessoires étant adaptés pour être chacun connecté ou non au module principal en fonction de phases respectives d'utilisation du dispositif.

Les accessoires comprennent une pluralité de modules fonctionnels, dont un ensemble de mélange (ou mélangeur), un ensemble d'injection (ou seringue), et un ensemble de transfert, sur lesquels on reviendra plus loin. Ils comprennent aussi un capuchon de fermeture amovible du module principal, et de préférence également un entonnoir, lequel n'est toutefois pas indispensable.

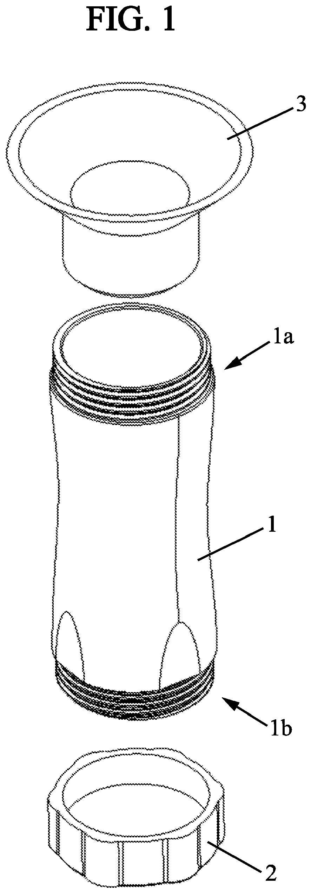

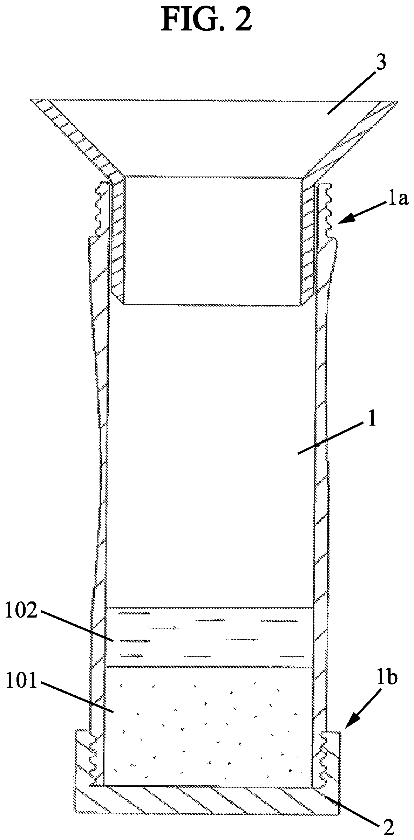

En référence aux Figures 1 et 2, le module principal 1 présente par exemple la forme d'un corps cylindrique creux, ayant une paroi interne, une extrémité proximale 1a et une extrémité distale 1b ouvertes. Dit autrement, le corps cylindrique 1 est ouvert à chacune de ses extrémités 1a et 1b.

L'extrémité distale 1b présente une ouverture de diamètre égal au diamètre du cylindre formé par la paroi intérieure du module principal 1.

Le mélange des composants du ciment osseux est réalisé dans ce corps cylindrique 1, qui est aussi appelé corps de mélange dans la suite.

Selon un mode de réalisation, le module principal 1 peut présenter un taraudage continu ou discontinu sur la paroi intérieure ou un filetage continu ou discontinu sur la paroi extérieure du module principal au niveau des extrémités proximale 1a et distale 1b.Ces filetages/taraudages servent à la connexion du module principal à un des accessoires, comme par exemple le capuchon de fermeture 2 représenté. A cet effet, le capuchon présente respectivement un filetage sa face extérieure ou un taraudage complémentaire sur sa face intérieure. Lorsqu'il est ainsi connecté à l'extrémité distale 1b, le capuchon 2 ferme le corps de mélange 1 au niveau de cette extrémité 1b, ainsi qu'il est montré à la Figure 2. La fermeture ainsi réalisée est étanche. Au besoin, un joint non représenté assure, ou contribue à cette étanchéité, par exemple un joint torique disposé dans le fond du capuchon 2 pour venir en appui contre le pourtour de l'extrémité distale 1b du corps de mélange 1.

Selon les phases d'utilisation, deux des accessoires du kit peuvent être simultanément connectés au corps de mélange 1, l'un à chacune de ses extrémités. Egalement, et ainsi qu'il sera exposé, plus d'un des accessoires peut se connecter à la même extrémité du corps de mélange 1, successivement.

Dans les modes de réalisation décrits ici, la connexion des accessoires du kit au module principal est réalisée par des filetages, de préférence avec des joints assurant l'étanchéité. Ceci n'est toutefois pas le seul moyen d'obtenir cette connexion. La connexion peut aussi être obtenue, par exemple, par encliquetage grâce à des moyens mutuellement complémentaires prévus sur le module principal et sur les accessoires.

Comme montré à la Figure 2, la partie cylindrique de l'entonnoir 3 peut être simplement engagée à l'intérieur du corps de mélange 1, au niveau de l'extrémité proximale 1a, sans connexion. A cet effet, le diamètre extérieur de la partie cylindrique de l'entonnoir 3 est légèrement inférieur au diamètre intérieur du corps de mélange 1, la partie tronconique et évasée de l'entonnoir 3 dépassant alors en dehors dudit corps de mélange 1, et assurant ainsi le maintien en position stable de l'entonnoir sous l'effet de la seule gravité lorsque le module principal est posé ou tenu à la verticale, comme montré à la Figure 2. En variante, l'entonnoir peut être adapté pour se connecter à l'extrémité proximale 1a du corps de mélange 1. A cet effet, la partie cylindrique de l'entonnoir peut, par exemple, présenter un filetage intérieur qui est complémentaire du filetage extérieur du corps de mélange 1 prévu au niveau de l'extrémité 1a pour la connexion d'autres accessoires au module principal.

Dans une phase de chargement des composants du ciment osseux dans le corps de mélange 1, en s'aidant ou non de l'entonnoir 3, l'utilisateur verse dans ledit corps, par son extrémité proximale 1a, les composants à mélanger pour former le ciment osseux. Il s'agit essentiellement, comme montré à la Figure 2, de la poudre 101 comprenant principalement des billes de pré-polymère et du liquide 102 contenant principalement le monomère.

Lors de cette opération, l'autre extrémité du corps de mélange 1, à savoir son extrémité distale 1b, est fermée par le capuchon 2.

L'utilisation de l'entonnoir évite toute perte de l'un ou de l'autre des produits à mélanger, qui pourrait affecter la composition finale du ciment une fois le mélange réalisé.

Après avoir versé les deux substances dans le corps de mélange 1, l'entonnoir 3 est enlevé pour laisser place à un autre accessoire, à savoir un ensemble de mélange.

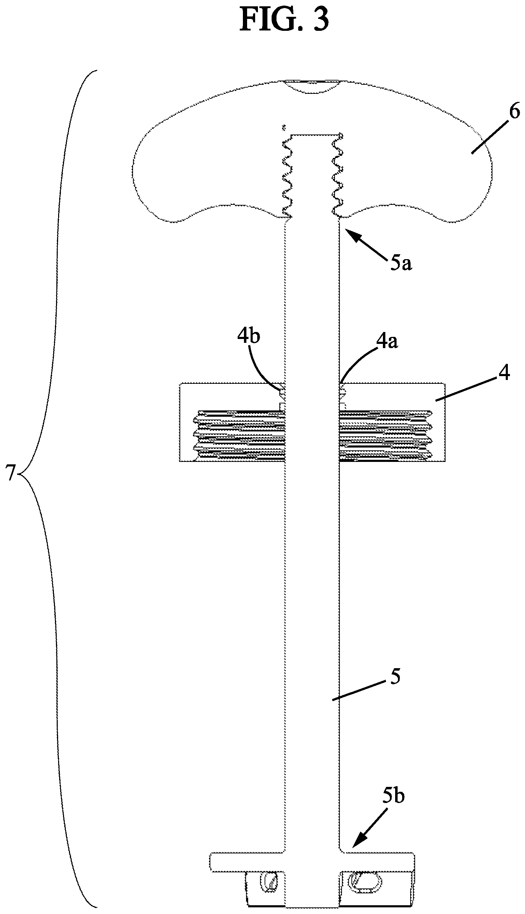

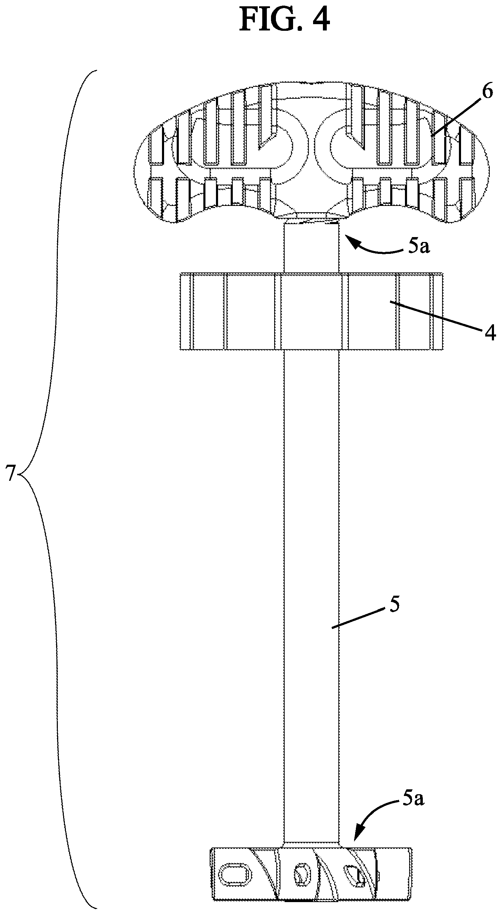

En référence aux Figures 3, 4 et 5, l'un des modules fonctionnels est en effet un ensemble de mélange, ou mélangeur 7. Le mélangeur 7 a pour fonction de permettre à l'utilisateur de réaliser le mélange des composants 101 et 102 du ciment osseux dans le corps du module principal 1. A cet effet, le mélangeur 7 est adapté pour être connecté, dans une phase de mélange, à l'extrémité proximale 1a du corps de mélange 1 alors que l'extrémité distale 1b dudit corps de mélange est toujours fermée par le capuchon de fermeture 2.

Le mélangeur 7 comprend un capuchon 4, et une tige d'actionnement 5 traversant ledit capuchon.

Le capuchon 4 est adapté pour fermer l'extrémité proximale 1a du corps de mélange 1 lorsque l'ensemble de mélange 7 est connecté audit module principal. Afin de réaliser cette connexion, le capuchon 4 peut comprendre un filetage intérieur complémentaire du filetage extérieur de l'extrémité proximale 1a du corps de mélange 1.

La tige d'actionnement 5 se présente comme un corps allongé, par exemple réalisée en matériau plein, d'aspect lisse à l'extérieur, de forme cylindrique, avec un diamètre substantiellement inférieur au diamètre interne du corps de mélange 1, et une longueur correspondant environ égale ou supérieure à la longueur dudit corps suivant son axe longitudinal.

Au niveau de l'une de ses extrémités 5b destinée à plonger dans le corps du module principal lorsque le mélangeur 7 est connecté au module principal 1, la tige d'actionnement 5 comprend des aubes de mélange, par exemple des pales ajourées comme montrées aux figures, ou non ajourées, qui participent au mélange des composants 101 et 102. Ces aubes peuvent être réalisées de matière avec la tige 5, ou sous la forme d'une ou plusieurs pièces agencées et fixées de manière à être solidaires de l'extrémité 5b de la tige 5 du mélangeur 7.

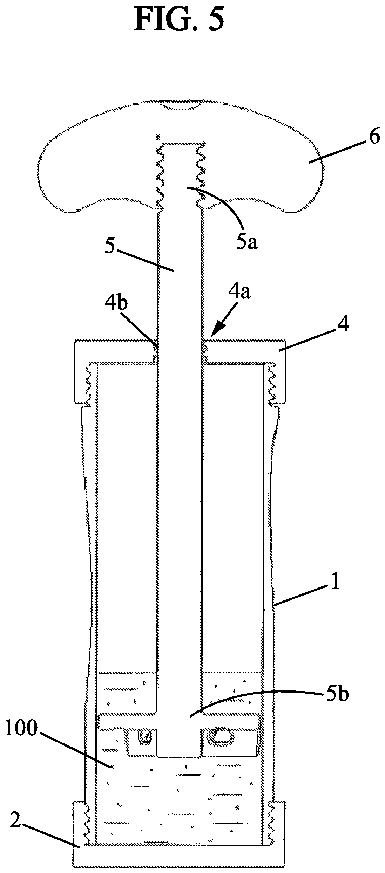

Au niveau de l'autre de ses extrémités 5a, opposée à l'extrémité 5b, la tige 5 est dotée d'une poignée de manoeuvre 6 permettant l'actionnement de ladite tige par l'utilisateur, depuis l'extérieur du module principal 1 lorsque le mélangeur 7 est connecté audit module principal. Dans l'exemple représenté aux figures, la poignée 6 est liée par un filetage intérieur à la tige 5 en son extrémité 5a qui comprend un filetage extérieur complémentaire. La poignée 6 permet, lors de la phase de réalisation du mélange, la préhension et la manoeuvre de l'ensemble de mélange par l'utilisateur afin de lui appliquer des mouvements de translation et de rotation autour de l'axe longitudinal de la tige 5. Ces mouvements sont répétés afin de mélanger la poudre 101 et le liquide 102, pendant le temps nécessaire et avec la dynamique nécessaire pour obtenir un ciment 100 homogène et prêt à l'emploi (Figure 5). Comme l'extrémité distale 1b présente une ouverture de diamètre égal au diamètre du cylindre formé par la paroi intérieure du module principal, le mélange complet de la poudre 101 et du liquide 102 et l'obtention d'un ciment homogène 100 sont facilités.

Le capuchon 4 du mélangeur 7 est similaire au capuchon de fermeture 2 qui a été décrit précédemment, sauf qu'il comprend, par exemple en son centre, un perçage 4a à travers lequel la tige 5 peut coulisser et tourner. Le perçage 4a peut être associé, par exemple, à des lamelles d'étanchéité 4b. Dans un exemple, deux telles lamelles permettent une étanchéité par contact avec la tige 5 lors de ses déplacements dans le corps de mélange 1, donc par rapport au capuchon 4 qui est lié audit corps de mélange. De cette façon, la tige 5 peut être entraînée en rotation étanche autour d'un axe longitudinal de ladite tige et en translation étanche suivant ledit axe longitudinal, en réponse à l'actionnement par l'utilisateur via la poignée 6. On notera que, lorsque l'ensemble de mélange 7 est connecté au module principal 1, l'axe longitudinal de sa tige 5 correspond aussi à l'axe longitudinal du corps cylindrique dudit module principal.

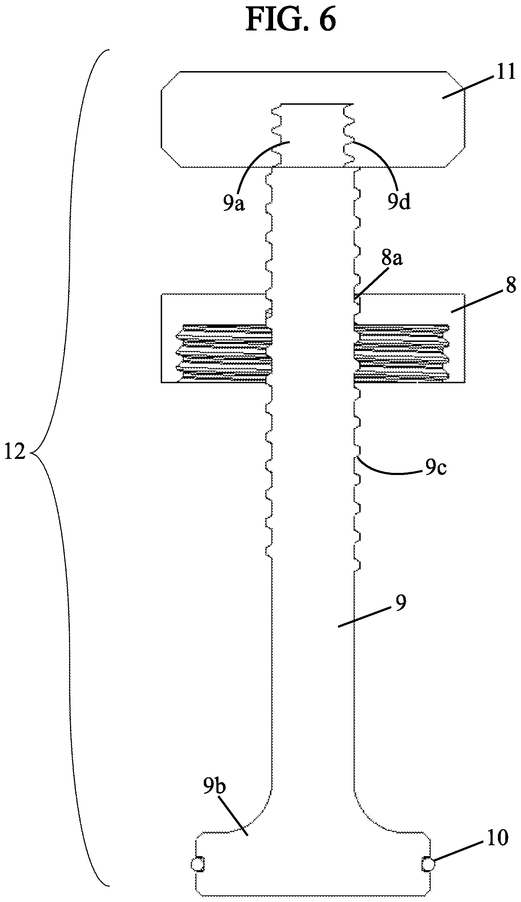

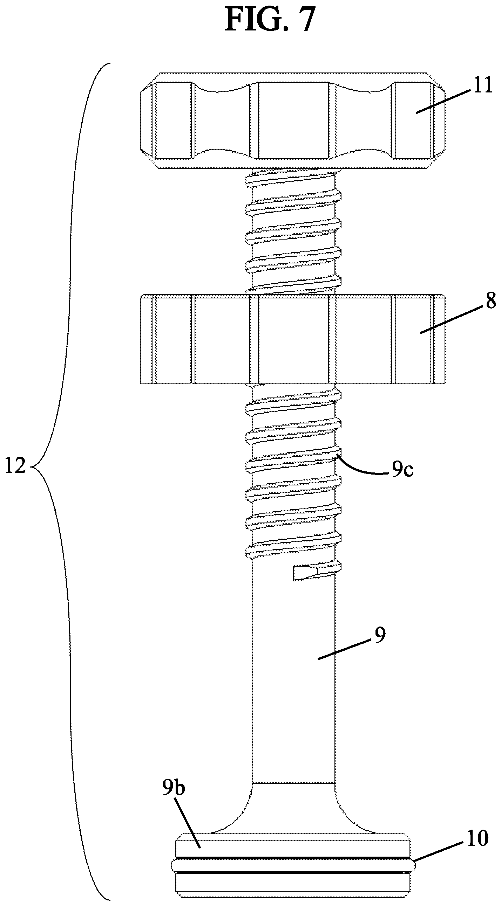

En référence aux Figures 6, 7, 8 ,9 et 10, un autre des modules fonctionnels est un ensemble de transfert, ou piston 9. Le piston 9 a pour fonction de permettre à l'utilisateur de réaliser le transfert du ciment 100 depuis le corps du module principal 1 vers un ensemble d'injection, ou seringue, qui est encore un autre accessoire sur lequel on reviendra plus loin. A cet effet, l'ensemble de transfert 12 est adapté pour être connecté, dans une phase de transfert venant ensuite de la phase de mélange, à l'extrémité proximale 1a du module principal, à la place de l'ensemble de mélange 7. Cette connexion est réalisée, dans un premier temps, alors que l'extrémité distale 1b du module de mélange 1 est toujours fermée par le capuchon de fermeture 2.

Dans le kit proposé, l'ensemble de transfert 12 est totalement distinct de l'ensemble de mélange 7, en ce sens qu'il n'a aucun élément en commun avec ce dernier. Dit autrement, la fonction de réalisation du mélange des composants du ciment jusqu'à l'obtention d'un ciment homogène prêt à l'emploi, d'une part, et la fonction de transfert du ciment depuis le corps principal dans lequel le mélange a été réalisé vers la seringue servant à l'injection du ciment, d'autre part, sont assurées par deux accessoires du kit distincts et indépendants l'un de l'autre, et également distincts de la seringue en elle-même. Elles sont successivement assurées dans des phases de fonctionnement également distinctes et indépendantes l'une de l'autre. Néanmoins le corps du module principal 1 est associé successivement à l'ensemble de mélange puis à l'ensemble de transfert. Egalement, lors de la phase d'injection, il participe à l'ergonomie du dispositif puisqu'il peut être utilisé comme moyen de préhension de la seringue par l'utilisateur.

Le piston 7 comprend un capuchon 8, et une tige d'actionnement 9 traversant ledit capuchon.

Comme le capuchon 4, le capuchon 8 est adapté pour fermer l'extrémité proximale 1a du corps du module principal lorsque l'ensemble de transfert 12 est connecté audit module principal. Afin de réaliser cette connexion, le capuchon 8 peut également comprendre un filetage intérieur complémentaire du filetage extérieur de l'extrémité proximale 1a du corps du module principal 1.

La tige d'actionnement 9 se présente comme un corps allongé, par exemple réalisée en matériau plein, de forme cylindrique, avec un diamètre substantiellement inférieur au diamètre interne du corps du module principal 1, et une longueur environ égale ou supérieure à la longueur dudit corps suivant son axe longitudinal. Contrairement à la tige 5 du mélangeur 7 qui est lisse à l'extérieur, la tige 9 du piston 12 est filetée sur une partie substantielle de sa longueur. Elle comprend en effet un filetage 9c.

Au niveau de l'une de ses extrémités 9b destinée à plonger dans le corps cylindrique du module principal 1lorsque le piston 12 est connecté audit module principal, la tige d'actionnement 9 comprend une tête de piston de forme cylindrique, d'un diamètre extérieur égal au diamètre intérieur du corps cylindrique du module principal 1. De manière plus générale, la tête de piston a une section de forme complémentaire de la forme de la section interne du corps du module principal 1. Comme l'extrémité distale 1b présente une ouverture de diamètre égal au diamètre du cylindre formé par la paroi intérieure du module principal 1, la forme de la tête de piston est avantageusement simple, c'est à dire plate. Cette forme simple est économique à réaliser et permet à l'utilisateur de réaliser facilement le transfert de l'ensemble du ciment 100 depuis le corps du module principal 1 vers l'ensemble d'injection. Dans l'exemple représenté, cette tête de piston est réalisée de matière avec la tige 9, et est donc également désignée ci-après par la référence 9b. Toutefois, elle peut aussi être réalisée sous la forme d'une ou plusieurs pièces agencées et fixées de manière à être solidaires de l'extrémité 9b de la tige 9 du piston 12. Si le corps cylindrique du module principal 1 présente une certaine élasticité, le diamètre extérieur de la tête 9b du piston 9 peut même être légèrement supérieur au diamètre intérieur dudit corps. La tête de piston 9b comprend une gorge annulaire où est logé un joint torique 10 adapté pour assurer l'étanchéité entre la tête de piston et la paroi intérieure du corps cylindrique du module principal 1.

Le capuchon 8 du piston 12 est similaire au capuchon 4 du mélangeur 7 qui a été décrit précédemment, sauf qu'il comprend, par exemple en son centre, un perçage taraudé 8a, complémentaire du filetage 9c du corps de la tige 9. L'engagement de ces deux filetages permet à la tige 9 et donc à la tête de piston 9a, d'avancer ou de reculer dans le corps cylindrique du module 1 en fonction du sens, i.e. vissage ou dévissage, dans lequel la tige 9 est actionnée, par exemple manoeuvrée par l'utilisateur. En d'autres termes, la rotation de la tige 9 du piston autour de son axe longitudinal est convertie en mouvement de translation suivant ledit axe, par l'effet des filetages précités. On notera que, lorsque le piston 12 est connecté au module principal 1, l'axe longitudinal de sa tige 9 correspond aussi à l'axe longitudinal du corps cylindrique dudit module principal.

Au niveau de l'autre de ses extrémités 9a, opposée à l'extrémité 9b, la tige 9 est dotée d'une poignée de manoeuvre 11 permettant l'actionnement de ladite tige par l'utilisateur, depuis l'extérieur du module principal 1 lorsque le piston 9 est connecté audit module principal. Dans l'exemple représenté aux figures, la poignée 11 est liée par un filetage intérieur à la tige 9 en son extrémité 9a qui comprend un filetage extérieur 9d complémentaire, lequel est de préférence distinct du filetage 9c du corps de la tige 9. D'autres moyens à la portée de l'Homme du métier, comme un encliquetage, un collage, un écrou, ou une ou plusieurs clavettes peuvent être prévus afin de garantir la bonne liaison de la poignée 11 avec la tige 9, même lorsqu'un mouvement de rotation de la tige 9 est imprimé par l'utilisateur par l'intermédiaire de la poignée 11 afin de faire progresser le piston 12.

La poignée 11 permet, lors de la phase de transfert, la préhension et la manoeuvre de l'ensemble de transfert par l'utilisateur afin de lui appliquer un mouvement de translation suivant l'axe longitudinal du corps du module principal 1, afin de pousser le ciment dans ce corps, ainsi qu'il va maintenant être explicité.

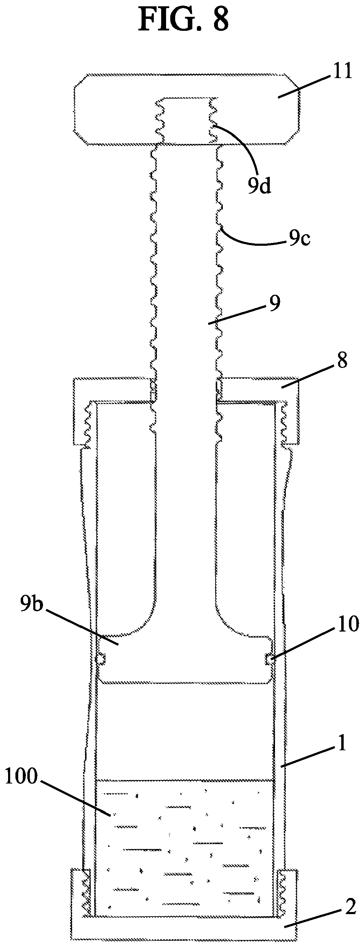

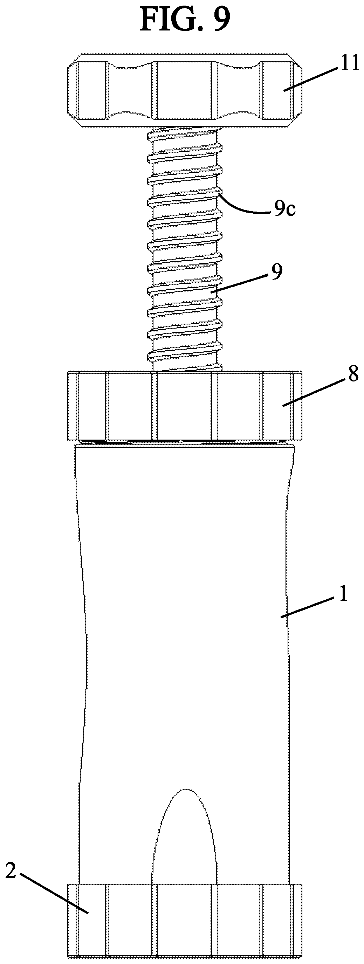

Une fois l'ensemble transfert 12 vissé au corps du module principal 1 comme montré aux Figures 8 et 9, le dispositif ainsi assemblé peut être retourné à 180° autour d'un axe horizontal imaginaire, c'est-à-dire de bas en haut, ou vice versa.

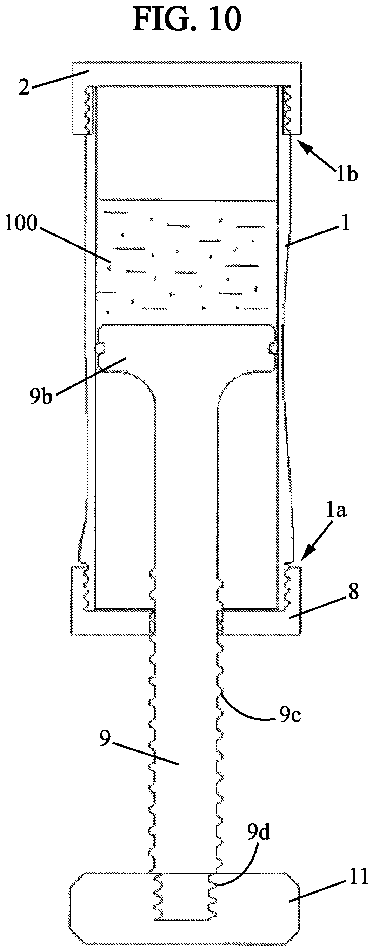

Le dispositif ainsi retourné comme montré à la Figure 10, le ciment 100 descend par gravité contre la tête de piston 9a. On peut alors enlever le capuchon de fermeture 2 de l'extrémité distale 1b du corps de mélange 1, pour poursuivre les opérations à l'aide du dernier accessoire du kit.

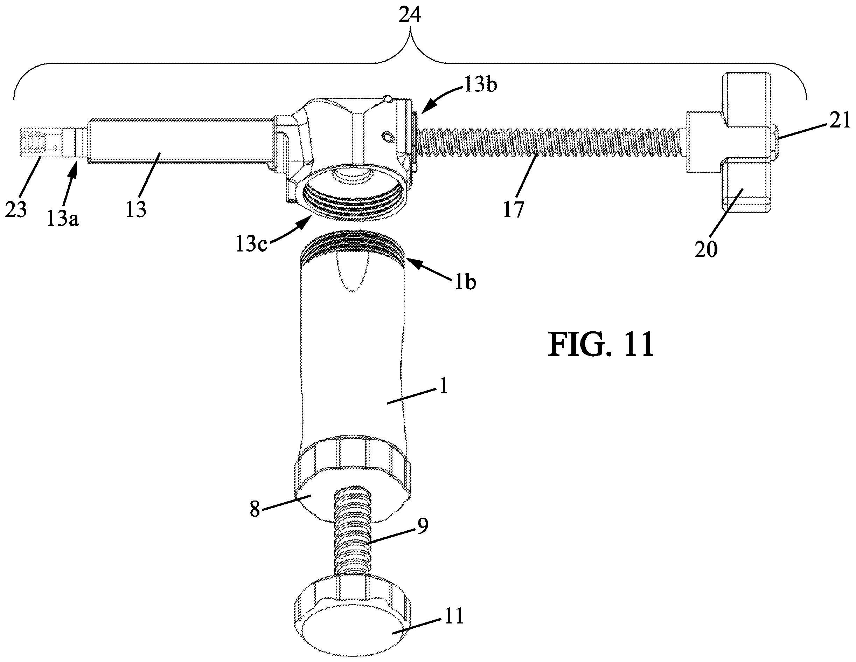

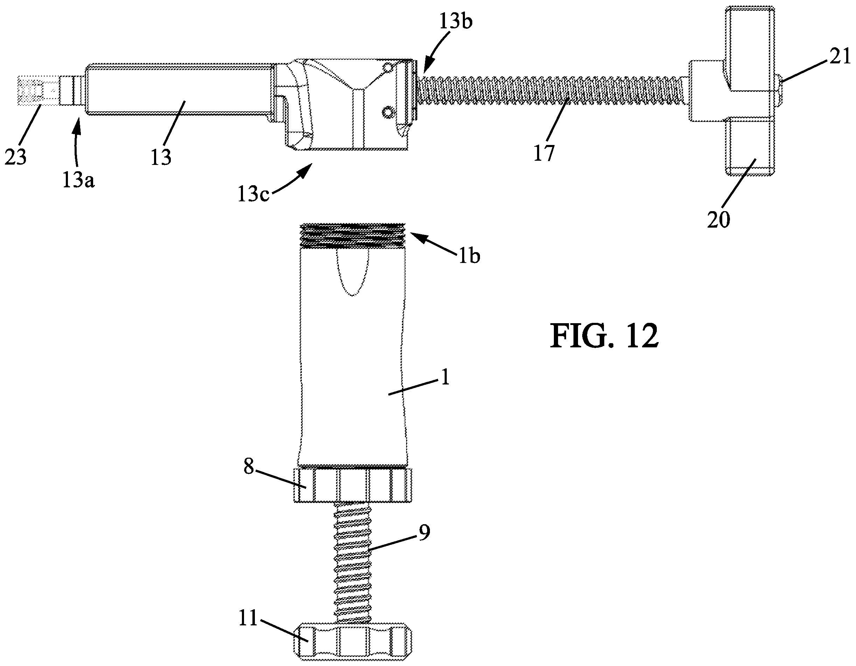

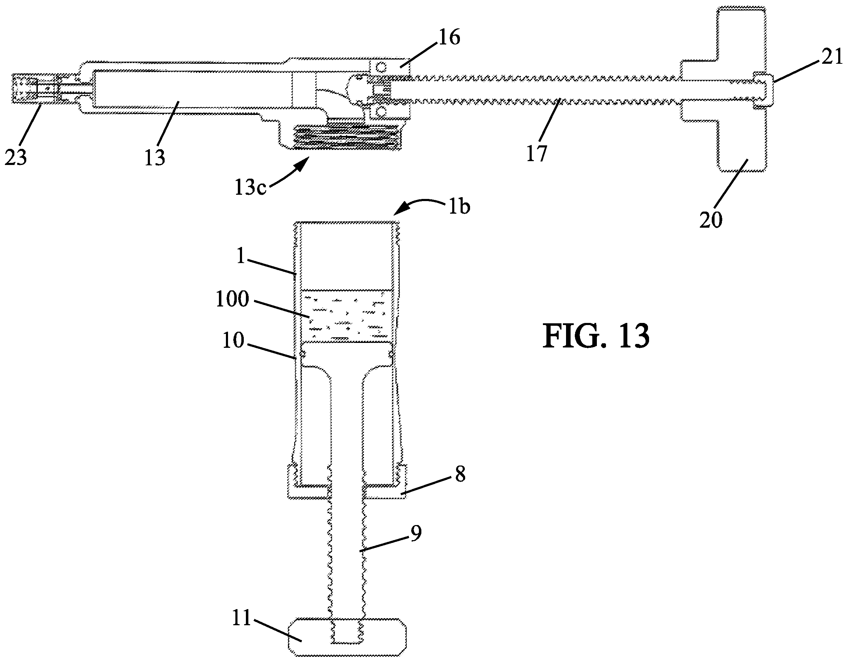

En effet, le kit comprend encore un autre module fonctionnel, en tant qu'accessoire du module principal, à savoir un ensemble d'injection ou seringue, qui va maintenant être décrit en référence aux Figures 11, 12, 13, 14 et 15.

Cet ensemble d'injection 24 est adapté pour être connecté, dans la phase de transfert ainsi que dans une phase d'injection venant ensuite de la phase de transfert, à l'extrémité distale 1b du module principal à la place du capuchon de fermeture 2. Ainsi connecté, il peut recevoir le ciment transféré depuis le module principal, pendant la phase de transfert. Ensuite, pendant la phase d'injection, il permet à l'utilisateur de réaliser l'injection du ciment osseux sur le site cible, par exemple la vertèbre à traiter.

Dans le mode de réalisation tel que représenté, l'ensemble d'injection 24 comprend un corps principal 13 de forme allongée, avec une chambre interne adaptée pour recevoir du ciment osseux prêt à être injecté.

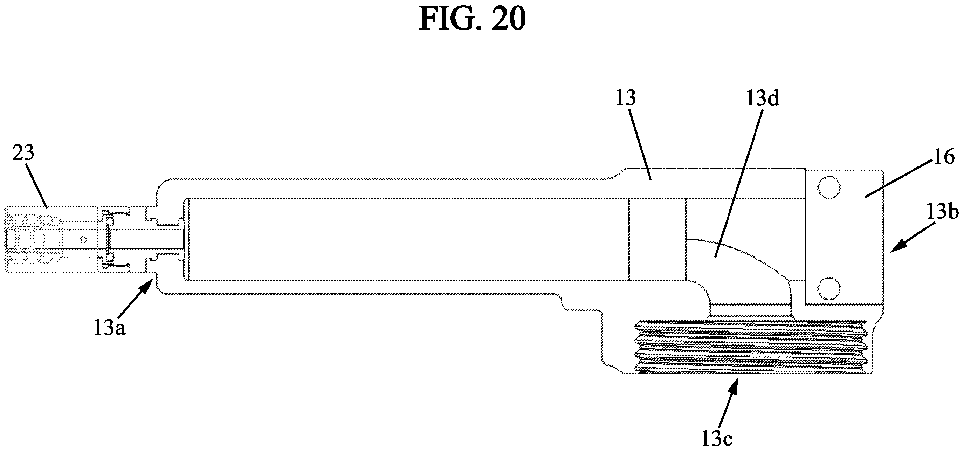

En référence à la Figure 20, le corps principal 13 de l'ensemble d'injection 24 possède trois ouvertures 13a, 13b et 13c. Les deux ouvertures 13a et 13b, qui sont au niveau des extrémités respectives du corps principal 13, présentent chacune un axe de passage orienté suivant l'axe longitudinal dudit corps 13, respectivement pour le ciment sortant dudit corps vers l'extérieur de la seringue, et pour la tige filetée du piston 22.

La troisième ouverture 13c présente un passage pour le ciment osseux dont l'axe est sécant, par exemple perpendiculairement, avec l'axe longitudinal du corps 13. L'ouverture 13c est une entrée de ciment osseux adaptée pour admettre le ciment dans la chambre interne dudit corps principal 13 depuis le corps de mélange du module principal 1. A cet effet, l'ouverture 13c du corps principal 13 est taraudée afin de se connecter par vissage à l'extrémité distale 1b au corps de mélange 1, comme montré sur les Figures 11, 12, 13, 14 et 15. L'admission du ciment osseux s'effectue lorsque l'ensemble de transfert 12 est connecté à l'extrémité proximale 1a du module principal 1, et est actionné par l'utilisateur afin de pousser le ciment 100 dans le corps de mélange vers l'extrémité distale 1b dudit module principal, alors que ladite extrémité distale est connectée à l'entrée 13c de la seringue comme il est dit ci-dessus.

Comme l'extrémité distale 1b présente une ouverture de diamètre égal au diamètre du cylindre formé par la paroi intérieure du module principal 1, la connexion entre ledit module principal et l'ensemble d'injection 24 est mécaniquement renforcée. Par conséquent, pendant les phases de transfert et d'injection, l'utilisateur peut avantageusement saisir le corps principal 1 comme une poignée, à la manière d'un pistolet, sans craindre que le dispositif de la présente invention ne se casse.

L'ouverture 13a est une sortie de ciment osseux adaptée pour, dans la phase d'injection de ciment osseux, dispenser le ciment osseux 100 à l'extérieur de l'ensemble d'injection 24, en réponse à une action de l'utilisateur. A cet effet, l'ouverture 13a du corps principal 13 est moulée avec une connexion 23 de type « luer-lock », cette connexion permettant de connecter la seringue à d'autres dispositifs médicaux qui peuvent être utilisés lors de l'intervention chirurgicale, notamment des buses d'injection de forme et de section variées.

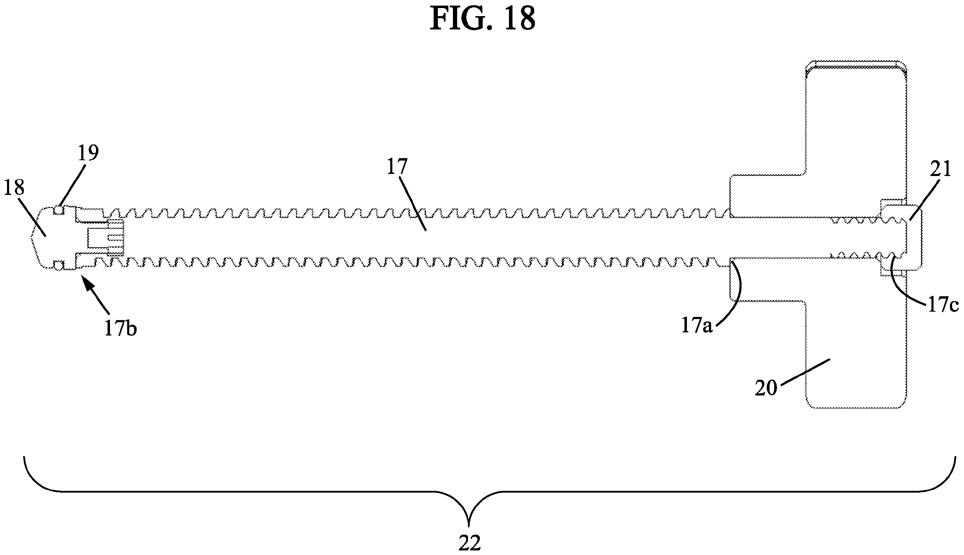

Enfin, l'ouverture 13b est adaptée pour le passage d'une tige de piston. En effet, la seringue 24 comprend aussi un piston 22 montré à la Figure 18. Le piston 22 comporte une tête de piston 18, ainsi qu'une tige 17 d'entraînement de ladite tête de piston, par exemple une tige filetée. La tête de piston 13 est adaptée pour pousser vers la sortie 13a le ciment osseux qui se trouve dans la chambre interne du corps principal 13 de la seringue. A cet effet, la tête de piston 18 est mue dans le corps principal 13, depuis l'ouverture 13b vers la sortie 13a, par l'intermédiaire de la tige d'entraînement 17 qui peut être actionnée par l'utilisateur depuis l'extérieur de l'ensemble d'injection 24. Cette manoeuvre provoque le déplacement la tête de piston 18 dans la chambre interne du corps principal 13 avec pour effet de pousser le ciment osseux vers la sortie 13a.

En référence en particulier à la Figure 18, la tige fileté 17 dispose à l'extrémité 17a du filetage qui est opposée à la tête de piston 18, d'un épaulement qui permet la mise en position d'une poignée de préhension et de manoeuvre 20. La poignée 20 peut être maintenue en position grâce à un écrou 21 vissé sur un autre filetage 17c à l'extrémité terminale de la tige 17. La tige 17 possède un autre épaulement en son extrémité 17b du côté de la tête de piston 18, cet épaulement accueillant un embout 18 qui est encliqueté dans la tige 17 contre cet épaulement. Cet embout 18 possède une gorge annulaire où est placé un joint torique 19 assurant l'étanchéité de la tête de piston 18 dans le corps principal 13 de la seringue 24.

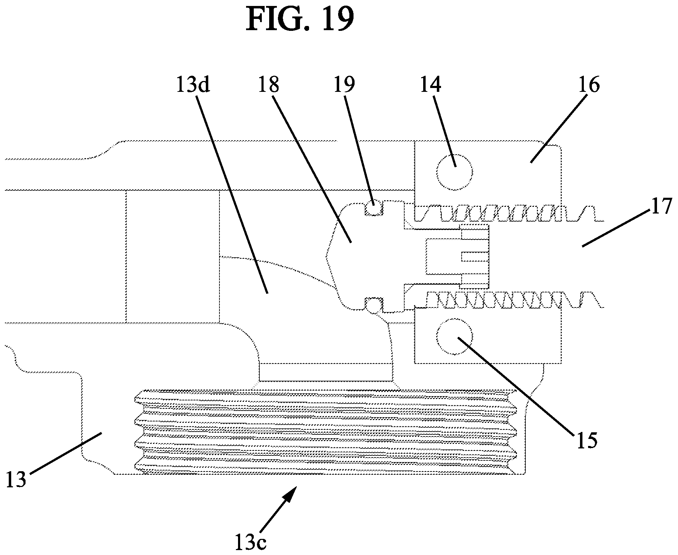

En référence à la Figure 19, l'ouverture 13b opposée à la sortie 13a suivant l'axe longitudinal du corps principal 13 de la seringue 24, reçoit une pièce intermédiaire 16 grâce à laquelle la tige filetée 17 du piston 22 est connectée audit corps principal de la seringue.

Dans l'exemple représenté, la pièce intermédiaire 16 comporte un perçage central taraudé, ce taraudage étant complémentaire du filetage de la tige 17. De plus, la pièce intermédiaire 16 est liée au corps principal 13 de la seringue 24 par deux tiges cylindriques, par exemple en acier, respectivement 14 et 15. D'autres forment de réalisation de cette liaison sont néanmoins possible, comme le collage, la soudure, l'encliquetage ou l'emboîtement à force, selon les matériaux dont sont réalisés les divers éléments du kit.

L'utilisation de la seringue 24 du dispositif est la suivante.

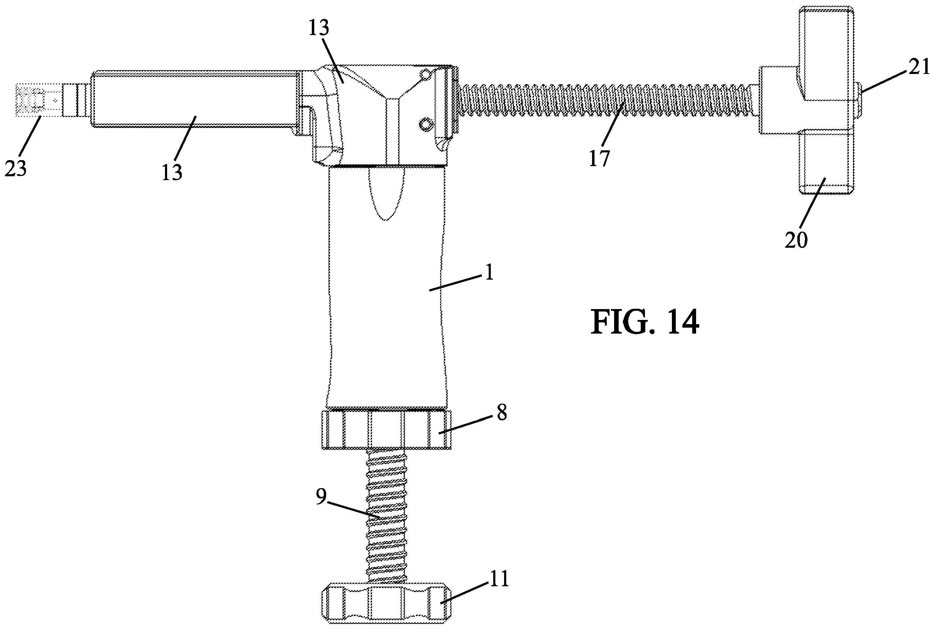

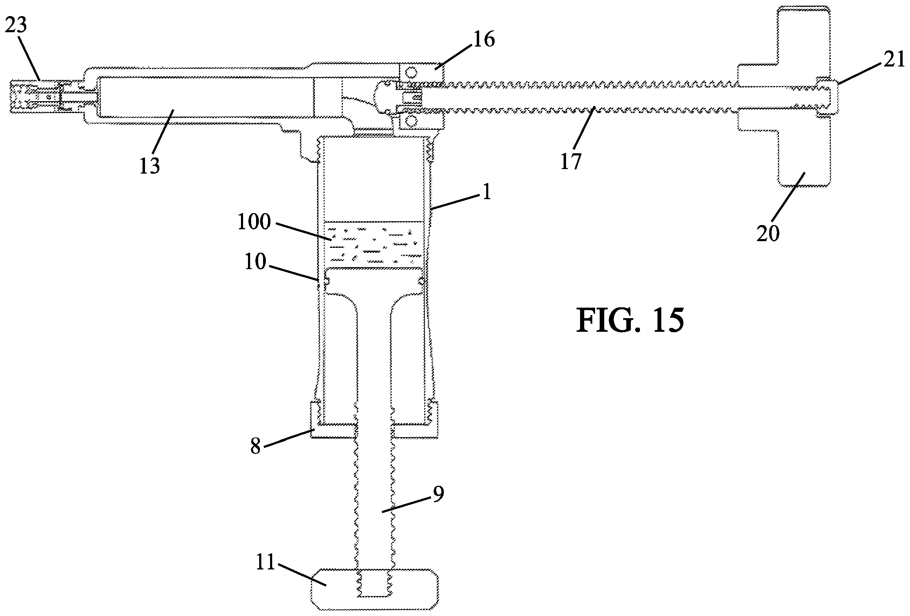

Une fois la seringue 24 et le corps de mélange 1 connectés ensemble, comme montré aux Figures 14 et 15, le ciment osseux peut être transféré depuis le corps de mélange 1 dans le corps principal 13 de la seringue, pendant la phase de transfert. Ce transfert est obtenu par vissage du piston 9 via la poignée 11 de l'ensemble de transfert 12, qui engendre le déplacement du piston 9 dans le corps de mélange 1 suivant l'axe longitudinal de ce dernier.

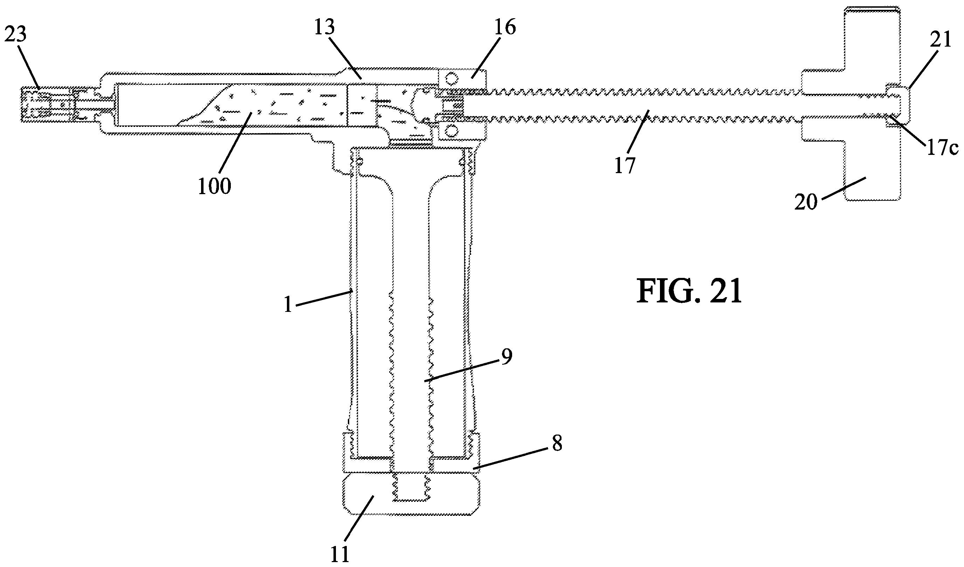

Une fois le ciment osseux intégralement transféré dans le volume du corps principal 13 de la seringue 24, comme montré à la Figure 21, le dispositif est prêt pour l'injection du ciment osseux dans le corps vertébral de la vertèbre à traiter.

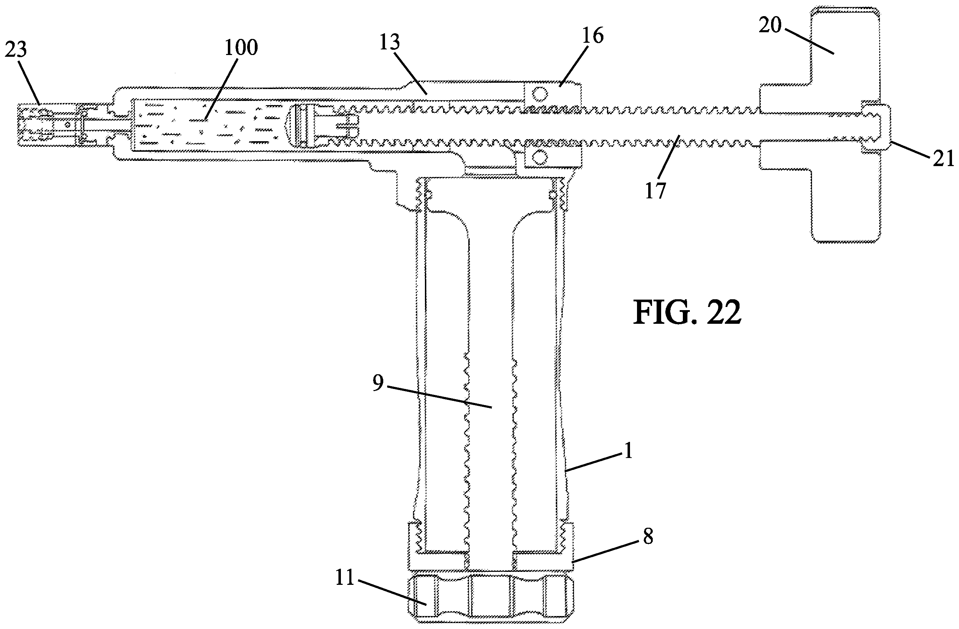

L'injection se fait alors, pendant la phase d'injection, en exerçant un mouvement de rotation sur la poignée 20, mouvement qui met en translation le piston 22 de la seringue. Ceci a pour effet de pousser le ciment osseux 100 dans le corps principal 13 de la seringue 24, suivant l'axe longitudinal de ce dernier, jusqu'à la sortie 13a ainsi qu'il est montré à la Figure 22.

Dans un mode de réalisation, illustré en particulier par les Figures 19, 20 et 21, l'ensemble d'injection comprend un canal coudé 13d, ayant une entrée couplée à l'entrée de ciment osseux 13c et une sortie débouchant dans la chambre interne du corps principal 13 en étant tournée vers la sortie de ciment osseux 13a. Ce canal 13d présente une ouverture au niveau du coude, ladite ouverture étant adaptée pour être traversée par la tête de piston 18 afin de permettre au ciment osseux 100 d'être poussé vers la sortie de ciment osseux 13a.

Dans des modes de réalisation, le corps de mélange du module principal 1 et/ou le corps principal 13 de l'ensemble d'injection 24 peuvent être réalisé en matériau transparent, comme du polycarbonate, afin de permettre à l'utilisateur d'observer le ciment. En particulier, l'utilisateur peut ainsi s'assurer, par un simple contrôle visuel, de l'homogénéité et de la viscosité du ciment, notamment pendant la phase de mélange, et de sa progression pendant la phase de transfert et pendant la phase d'injection, respectivement.

La présente invention a été décrite et illustrée dans la présente description détaillée et dans les Figures. La présente invention ne se limite pas aux formes de réalisation présentées. D'autres variantes et modes de réalisation peuvent être déduits et mis en oeuvre par la personne du métier à la lecture de la présente description et des Figures annexées.

Dans les revendications, le terme "comporter" n'exclut pas d'autres éléments ou d'autres étapes. Les différentes caractéristiques présentées et/ou revendiquées peuvent être avantageusement combinées. Leur présence dans la description ou dans des revendications dépendantes différentes, n'excluent pas cette possibilité. Les signes de référence ne sauraient être compris comme limitant la portée de l'invention.

## Revendications

1. Dispositif en kit pour le mélange et l'injection d'un ciment osseux, comprenant un module principal (1), d'une part, et des accessoires comprenant un capuchon de fermeture amovible (2) et une pluralité de modules fonctionnels (7,12,24), d'autre part, lesdits accessoires étant adaptés pour être chacun connecté ou non au module principal en fonction de phases respectives d'utilisation du dispositif, dans lequel :
- le module principal est un corps cylindrique creux ayant une paroi interne, une extrémité proximale (1a) et une extrémité distale (1b) ouvertes, adaptées pour être connectées chacune à un ou plusieurs des accessoires du dispositif, selon les phases d'utilisation, l'extrémité distale (1b) comprenant une ouverture présentant un diamètre égale au diamètre du cylindre formé par la paroi interne du corps cylindrique creux ;
- un des modules fonctionnels est un ensemble de mélange (7), adapté pour être connecté, dans une phase de mélange, à l'extrémité proximale (1a) du module principal alors que l'extrémité distale (1b) dudit module principal est fermée par le capuchon de fermeture (2), et pour permettre à un utilisateur de réaliser le mélange d'au moins deux composés du ciment osseux dans le corps du module principal (1) ;
- un autre des modules fonctionnels est un ensemble d'injection (24), adapté pour être connecté, dans une phase de transfert venant ensuite de la phase de mélange ainsi que dans une phase d'injection venant ensuite de ladite phase de transfert, à l'extrémité distale (1b) du module principal à la place du capuchon de fermeture, et pour permettre à l'utilisateur de réaliser l'injection du ciment osseux ;
- un autre encore des modules fonctionnels, distinct de l'ensemble de mélange et sans aucun élément commun avec l'ensemble de mélange, est un ensemble de transfert (12), adapté pour être connecté, dans la phase de transfert, à l'extrémité proximale (1a) du module principal à la place de l'ensemble de mélange et pour permettre à l'utilisateur de transférer du ciment osseux depuis le corps du module principal vers l'ensemble d'injection.

2. Dispositif selon la revendication 1, dans lequel les accessoires comprennent en outre un entonnoir (3), adapté pour permettre, avant la phase de mélange, le versement via l'extrémité proximale (1a) du module principal des composés du ciment osseux à mélanger dans le corps dudit module principal, alors que l'extrémité distale (1b) du module principal est fermée par le capuchon de fermeture (2).

3. Dispositif selon la revendication 1 ou la revendication 2, dans lequel l'ensemble de mélange (7) et l'ensemble de transfert (12) comprennent chacun un capuchon (4,8) et une tige d'actionnement (5,9) traversant ledit capuchon, le capuchon de chacun des ensembles étant adapté pour fermer l'extrémité proximale (1a) du corps du module principal lorsque l'ensemble correspondant est connecté audit module principal.

4. Dispositif selon la revendication 3, dans lequel :
- la tige d'actionnement (5) de l'ensemble de mélange (7) comprend des aubes de mélange au niveau d'une première extrémité (5b) de ladite tige destinée à plonger dans le corps du module principal, ainsi qu'une poignée de préhension (6) au niveau d'une seconde extrémité (5a) de ladite tige permettant l'actionnement par l'utilisateur depuis l'extérieur du module principal ; et,
- le capuchon de l'ensemble de mélange comprend un perçage (4a) associé à des lamelles d'étanchéité, à travers lequel la tige de l'ensemble de mélange peut être entraînée en rotation étanche autour d'un axe longitudinal de ladite tige et en translation étanche suivant un axe longitudinal du corps cylindrique du module principal, en réponse à l'actionnement par l'utilisateur via la poignée de ladite tige.

5. Dispositif selon la revendication 3 ou la revendication 4, dans lequel :
- la tige d'actionnement (9) de l'ensemble de transfert (12) est filetée sur au moins une partie de sa longueur et comprend une tête de piston au niveau d'une première extrémité (9b) de ladite tige destinée à plonger dans le corps du module principal, ainsi qu'une poignée de préhension (11) au niveau d'une seconde extrémité (9a) de ladite tige permettant l'actionnement par l'utilisateur depuis l'extérieur du module principal ;
- la tête de piston a une section de forme complémentaire de la forme de la section interne du corps du module principal et est dotée d'une gorge périphérique recevant un joint d'étanchéité ; et,
- le capuchon (8) de l'ensemble de transfert comprend un perçage taraudé pour lier la tige d'actionnement (9) de l'ensemble de transfert (12) au capuchon dudit ensemble, en coopération avec le filetage de ladite tige, et à travers lequel ladite tige peut être entraînée par vissage/dévissage pour manoeuvrer le piston en réponse à l'actionnement par l'utilisateur via la poignée de ladite tige.

6. Dispositif selon l'une quelconque des revendications 1 à 5, dans lequel l'ensemble d'injection comprend :
- un corps allongé (13) avec une chambre interne adaptée pour recevoir du ciment osseux prêt à être injecté ;
- une entrée de ciment osseux (13c), adaptée pour admettre du ciment osseux dans la chambre interne depuis le corps du module principal (1), lorsque l'ensemble de transfert (12) est connecté à l'extrémité distale (1b) dudit module principal et est actionné par l'utilisateur ;
- une tête de piston (18) ainsi qu'une tige d'entraînement (17) de ladite tête de piston qui peut être actionnée par l'utilisateur depuis l'extérieur de l'ensemble d'injection (24) pour manoeuvrer ladite tête de piston dans la chambre interne du corps allongé (13) afin de pousser le ciment osseux (100) ; et,
- une sortie de ciment osseux (23), adaptée pour dispenser le ciment osseux à l'extérieur de l'ensemble d'injection en réponse à une action de l'utilisateur via la tige d'entraînement (17) de la tête de piston (18).

7. Dispositif selon la revendication 6, dans lequel l'ensemble d'injection comprend un canal coudé (19d), ayant une entrée couplée à l'entrée de ciment osseux (13c) et une sortie débouchant dans la chambre interne du corps allongé (13) en étant tournée vers la sortie de ciment osseux (23), ainsi qu'une ouverture ou niveau du coude du canal, ladite ouverture étant adaptée pour être traversée par la tête de piston (18) afin de permettre au ciment osseux (100) d'être poussé vers ladite sortie de ciment osseux (23).

8. Dispositif selon l'une quelconque des revendications 6 et 7, dans lequel le module principal (1) comprend, au niveau de son extrémité distale (1b), un filetage apte à coopérer avec un filetage complémentaire de l'ensemble d'injection (24) au niveau de l'entrée (13c) dudit ensemble, pour assurer la connexion de l'ensemble d'injection au module principal (1).

9. Dispositif selon l'une quelconque des revendications 3 à 7, dans lequel le module principal (1) comprend, au niveau de son extrémité proximale (1a) un filetage apte à coopérer avec un filetage complémentaire des capuchons respectifs (4,8) de l'ensemble de mélange (7) et de l'ensemble de transfert (12).

10. Dispositif selon l'une quelconque des revendications 1 à 9, dans lequel l'extrémité distale (1b) présente une ouverture de diamètre égal au diamètre d'un cylindre formé par une paroi intérieure du module principal (1).

11. Procédé de mélange et d'injection d'un ciment osseux à l'aide d'un dispositif en kit comprenant un module principal (1) ayant la forme d'un corps cylindrique creux ayant une paroi interne, une extrémité proximale (1a) et une extrémité distale (1b) ouvertes d'une part et présentant une ouverture dont le diamètre est égale au diamètre du cylindre formé par la paroi interne du corps cylindrique creux (1), et des accessoires comprenant un capuchon de fermeture amovible (2) et une pluralité de modules fonctionnels (7,12,24), d'autre part, le procédé comprenant les étapes consistant à:
a) dans une phase de mélange, connecter un des modules fonctionnels ou ensemble de mélange (7) à l'extrémité proximale (1a) du module principal alors que l'extrémité distale (1b) dudit module principal est fermée par le capuchon de fermeture (2), et réaliser le mélange d'au moins deux composés du ciment osseux dans le corps du module principal (1) ;
b) dans une phase de transfert venant ensuite de la phase de mélange :
b1) connecter un autre des modules fonctionnels ou ensemble de transfert (12), distinct de l'ensemble de mélange et sans aucun élément commun avec l'ensemble de mélange, à l'extrémité proximale (1a) du module principal à la place de l'ensemble de mélange, et
b2) connecter un autre encore des modules fonctionnels ou ensemble d'injection (24) à l'extrémité distale (1b) du module principal à la place du capuchon de fermeture,
b3) transférer du ciment osseux depuis le corps du module principal vers l'ensemble d'injection ; et
c) dans une phase d'injection venant ensuite de la phase de transfert, réaliser l'injection du ciment osseux.

## Patentansprüche

1. Vorrichtung als Bausatz für das Mischen und Einspritzen eines Knochenzements, umfassend einerseits ein Hauptmodul (1) und andererseits Zubehörteile, umfassend eine abnehmbare Verschlusskappe (2) und eine Vielzahl von Funktionsmodulen (7, 12, 24), wobei die Zubehörteile dazu ausgelegt sind, je nach den jeweiligen Verwendungsphasen der Vorrichtung jeweils mit dem Hauptmodul verbunden zu sein oder nicht, wobei:
- das Hauptmodul ein hohler zylindrischer Körper ist, der eine Innenwand, ein offenes proximales Ende (1a) und ein offenes distales Ende (1b) aufweist, die dazu ausgelegt sind, entsprechend den Verwendungsphasen jeweils mit einem oder mehreren der Zubehörteile der Vorrichtung verbunden zu werden, wobei das distale Ende (1b) eine Öffnung umfasst, die einen Durchmesser aufweist, der gleich dem Durchmesser des von der Innenwand des hohlen zylindrischen Körpers gebildeten Zylinders ist;
- eines der Funktionsmodule eine Mischanordnung (7) ist, die dazu ausgelegt ist, in einer Mischphase mit dem proximalen Ende (1a) des Hauptmoduls verbunden zu sein, während das distale Ende (1b) des Hauptmoduls von der Verschlusskappe (2) verschlossen ist, und um es einem Benutzer zu ermöglichen, das Mischen von mindestens zwei Bestandteilen des Knochenzements im Körper des Hauptmoduls (1) durchzuführen;
- ein anderes der Funktionsmodule eine Einspritzanordnung (24) ist, die dazu ausgelegt ist, in einer der Mischphase folgenden Übertragungsphase sowie in einer der Übertragungsphase folgenden Einspritzphase, anstelle der Verschlusskappe mit dem distalen Ende (1b) des Hauptmoduls verbunden zu sein und es dem Benutzer zu ermöglichen, das Einspritzen des Knochenzements durchzuführen;
- noch ein anderes der Funktionsmodule, das getrennt von der Mischanordnung ist und kein gemeinsames Element mit der Mischanordnung aufweist, eine Übertragungsanordnung (12) ist, die dazu ausgelegt ist, in der Übertragungsphase anstelle der Mischanordnung mit dem proximalen Ende (1a) des Hauptmoduls verbunden zu sein und um es dem Benutzer zu ermöglichen, Knochenzement vom Körper des Hauptmoduls in Richtung der Einspritzanordnung zu übertragen.

2. Vorrichtung nach Anspruch 1, wobei die Zubehörteile unter anderem einen Trichter (3) umfassen, der dazu ausgelegt ist, vor der Mischphase das Einfüllen von Bestandteilen des Knochenzements über das proximale Ende (1a) des Hauptmoduls zu ermöglichen, die im Körper des Hauptmoduls zu vermischen sind, während das distale Ende (1b) des Hauptmoduls durch die Verschlusskappe (2) verschlossen ist.

3. Vorrichtung nach Anspruch 1 oder Anspruch 2, wobei die Mischanordnung (7) und die Übertragungsanordnung (12) jeweils eine Kappe (4, 8) und eine Betätigungsstange (5, 9), die durch die Kappe verläuft, umfassen, wobei die Kappe jeder der Anordnungen dazu ausgelegt ist, das proximale Ende (1a) des Körpers des Hauptmoduls zu verschließen, wenn die entsprechende Anordnung mit dem Hauptmodul verbunden ist.

4. Vorrichtung nach Anspruch 3, wobei:
- die Betätigungsstange (5) der Mischanordnung (7) an einem ersten Ende (5b) der Stange, die dazu bestimmt ist, in den Körper des Hauptmoduls einzutauchen, Mischschaufeln sowie an einem zweiten Endes (5a) der Stange einen Handgriff (6), der die Betätigung von außerhalb des Hauptmoduls durch den Benutzer ermöglicht, umfasst; und
- die Kappe der Mischanordnung eine mit Dichtungslamellen zugeordnete Bohrung (4a) umfasst, durch die die Stange der Mischanordnung als Reaktion auf die Betätigung über den Griff der Stange durch den Benutzer, abgedichtet um eine Längsachse der Stange, drehend und, abgedichtet entlang einer Längsachse des zylindrischen Körpers des Hauptmoduls translatorisch angetrieben werden kann.

5. Vorrichtung nach Anspruch 3 oder Anspruch 4, wobei:
die Betätigungsstange (9) der Übertragungsanordnung (12) zumindest auf einem Teil ihrer Länge mit einem Gewinde versehen ist und an einem ersten Ende (9b) der Stange, das dazu bestimmt ist, in den Körper des Hauptmoduls zu einzutauchen, einen Kolbenkopf sowie an einem zweiten Ende (9a) der Stange einen Handgriff (11), der die Betätigung von außerhalb des Hauptmoduls durch den Benutzer ermöglicht, umfasst;
- wobei der Kolbenkopf einen Abschnitt mit zur Form des inneren Abschnitts des Körpers des Hauptmoduls komplementärer Form aufweist und mit einer umlaufenden Nut versehen ist, die eine Dichtung aufnimmt; und
- die Kappe (8) der Übertragungsanordnung eine Gewindebohrung umfasst, um die Betätigungsstange (9) der Übertragungsanordnung (12), zusammenwirkend mit dem Gewinde der Stange, mit der Kappe der Anordnung zu koppeln, und durch welche hindurch die Stange durch Einschrauben/Ausschrauben angetrieben werden kann, um den Kolben als Reaktion auf die Betätigung über den Griff der Stange durch den Benutzer zu führen.

6. Vorrichtung nach einem der Ansprüche 1 bis 5, wobei die Einspritzanordnung umfasst:
- einen länglichen Körper (13) mit einer inneren Kammer, die dazu ausgelegt ist, zum Eingespritztwerden bereiten Knochenzement aufzunehmen;
- einen Knochenzementeinlass (13c), der dazu ausgelegt ist, Knochenzement aus dem Körper des Hauptmoduls (1) in die innere Kammer einzulassen, wenn die Übertragungsanordnung (12) mit dem distalen Ende (1b) des Hauptmoduls verbunden ist und vom Benutzer betätigt wird;
- einen Kolbenkopf (18) sowie eine Antriebsstange (17) des Kolbenkopfes, die vom Benutzer von außerhalb der Einspritzanordnung (24) betätigt werden kann, um den Kolbenkopf in die innere Kammer des länglichen Körpers (13) zu führen, um den Knochenzement (100) zu schieben; und
- einen Knochenzementauslass (23), der dazu ausgelegt ist, den Knochenzement als Reaktion auf eine Aktion des Benutzers über die Antriebsstange (17) des Kolbenkopfes (18) außerhalb der Einspritzanordnung auszugeben.

7. Vorrichtung nach Anspruch 6, wobei die Einspritzanordnung einen abgeknickten Kanal (19d) umfasst, der einen Einlass, der mit dem Knochenzementeinlass (13c) gekoppelt ist, und einen Auslass, der in die innere Kammer des länglichen Körpers (13) mündet und in Richtung des Knochenzementauslasses (23) gewandt ist, sowie eine Öffnung auf Höhe des Knicks des Kanals umfasst, wobei die Öffnung dazu ausgelegt ist, vom Kolbenkopf (18) durchquert zu werden, um es Knochenzement (100) zu ermöglichen, in Richtung des Knochenzementauslasses (23) geschoben zu werden.

8. Vorrichtung nach einem der Ansprüche 6 und 7, wobei das Hauptmodul (1) an seinem distalen Ende (1b) ein Gewinde umfasst, das in der Lage ist, mit einem komplementären Gewinde der Einspritzanordnung (24) am Einlass (13c) der Anordnung zusammenzuwirken, um die Verbindung der Einspritzanordnung mit dem Hauptmodul (1) sicherzustellen.

9. Vorrichtung nach einem der Ansprüche 3 bis 7, wobei das Hauptmodul (1) an seinem proximalen Endes (1a) ein Gewinde umfasst, das in der Lage ist, mit einem komplementären Gewinde der jeweiligen Kappe (4, 8) der Mischanordnung (7) und der Übertragungsanordnung (12) zusammenzuwirken.

10. Vorrichtung nach einem der Ansprüche 1 bis 9, wobei das distale Ende (1b) eine Öffnung aufweist, deren Durchmesser gleich dem Durchmesser eines von einer Innenwand des Hauptmoduls (1) gebildeten Zylinders ist.

11. Verfahren zum Mischen und Einspritzen eines Knochenzements mithilfe einer Vorrichtung als Bausatz, die einerseits ein Hauptmodul (1), das die Form eines hohlen zylindrischen Körpers aufweist, der eine Innenwand, ein offenes proximales Ende (1a) und ein offenes distales Ende (1b) aufweist, und eine Öffnung aufweist, deren Durchmesser gleich dem Durchmesser des von der Innenwand des hohlen zylindrischen Körpers (1) gebildeten Zylinders ist, und andererseits Zubehörteile umfasst, die eine abnehmbare Verschlusskappe (2) und eine Vielzahl von Funktionsmodulen (7, 12, 24) umfassen, wobei das Verfahren die Schritte umfasst, die darin bestehen:
a) Verbinden eines der Funktionsmodule oder der Mischanordnung (7) mit dem proximalen Ende (1a) des Hauptmoduls in einer Mischphase, während das distale Ende (1b) des Hauptmoduls von der Verschlusskappe (2) verschlossen ist, und Durchführen des Mischens von mindestens zwei Bestandteilen des Knochenzements im Körper des Hauptmoduls (1);
b) in einer auf die Mischphase folgenden Übertragungsphase:
b1) Verbinden eines anderen der Funktionsmodule oder der Übertragungsanordnung (12), das getrennt von der Mischanordnung ist und kein gemeinsames Element mit der Mischanordnung aufweist, anstelle der Mischanordnung mit dem proximalen Ende (1a) des Hauptmoduls, und
b2) Verbinden noch eines anderen der Funktionsmodule oder der Einspritzanordnung (24) anstelle der Verschlusskappe mit dem distalen Ende (1b) des Hauptmoduls,
b3) Übertragen des Knochenzements aus dem Körper des Hauptmoduls in Richtung der Einspritzanordnung; und
c) Durchführen des Einspritzens des Knochenzements in einer der Übertragungsphase folgenden Einspritzphase.

## Claims

1. Kit-type device for mixing and injecting a bone cement, comprising a main module (1), on the one hand, and accessories comprising a removable closing cap (2) and a plurality of functional modules (7, 12, 24), on the other hand, said accessories being adapted to be each connected or not to the main module according to respective phases of use of the device, wherein:
- the main module is a hollow cylindrical body having an internal wall, an open proximal end (1a) and an open distal end (1b), adapted to be each connected to one or a plurality of the accessories of the device, according to the phases of use, the distal end (1b) comprising an opening with a diameter equal to the diameter of the cylinder formed by the internal wall of the hollow cylindrical body;
- one of the functional modules is a mixing unit (7), adapted to be connected, in a mixing phase, to the proximal end (1a) of the main module while the distal end (1b) of said main module is closed by the closing cap (2), and to enable a user to mix at least two compounds of the bone cement in the body of the main module (1);
- a further functional module is an injection unit (24), adapted to be connected, in a transfer phase following the mixing phase as well as in an injection phase following said transfer phase, to the distal end (1b) of the main module instead of the closing cap, and to enable the user to inject the bone cement;
- a further still functional module, distinct from the mixing unit and with no common element with the mixing unit, is a transfer unit (12), adapted to be connected, in the transfer phase, to the proximal end (1a) of the main module instead of the mixing unit and to enable the user to transfer bone cement from the body of the main module to the injection unit.

2. Device according to claim 1, wherein the accessories further comprise a funnel (3), adapted to enable, before the mixing phase, pouring via the proximal end (1a) of the main module of the compounds of the bone cement to be mixed in the body of said main module, while the distal end (1b) of the main module is closed by the closing cap (2).

3. Device according to claim 1 or claim 2, wherein the mixing unit (7) and the transfer unit (12) each comprise a cap (4, 8) and an actuation rod (5, 9) passing through said cap, the cap of each of the units being adapted to close the proximal end (1a) of the body of the main module when the corresponding unit is connected to said main module.

4. Device according to claim 3, wherein:
- the actuation rod (5) of the mixing unit (7) comprises mixing blades at a first end (5b) of said rod intended to plunge into the body of the main module, as well as a gripping handle (6) at a second end (5a) of said rod enabling actuation by the user from outside the main module; and,
- the cap of the mixing unit comprises a hole (4a) associated with sealing strips, through which the rod of the mixing unit can be actuated in water tight rotation about a longitudinal axis of said rod and in water tight translation along a longitudinal axis of the cylindrical body of the main module, in response to the actuation by the user via the handle of said rod.

5. Device according to claim 3 or claim 4, wherein:
- the actuation rod (9) of the transfer unit (12) is threaded on at least a part of the length thereof and comprises a piston head at a first end (9b) of said rod intended to plunge into the body of the main module, as well as a gripping handle (11) at a second end (9a) of said rod enabling actuation by the user from outside the main module;
- the piston head has a cross-section with a shape complementary with the shape of the internal cross-section of the body of the main module and is provided with a peripheral groove receiving a seal; and
- the cap (8) of the transfer unit comprises a threaded hole to connect the actuation rod (9) of the transfer unit (12) to the cap of said unit, when engaging with the screw thread of said rod, and whereby said rod can be actuated by screwing/unscrewing to operate the piston in response to the actuation by the user via the handle of said rod.

6. Device according to any one of claims 1 to 5, wherein the injection unit comprises:
- an elongated body (13) with an internal chamber adapted to receive bone cement ready for injection;
- a bone cement inlet (13c), adapted to admit bone cement into the internal chamber from the body of the main module (1), when the transfer unit (12) is connected to the distal end (1b) of said main module and is actuated by the user;
- a piston head (18) as well as an actuation rod (17) of said piston head which can be actuated by the user from outside the injection unit (24) to operate said piston head in the internal chamber of the elongated body (13) in order to push the bone cement (100); and,
- a bone cement outlet (23), adapted to dispense the bone cement outside the injection unit in response to an action by the user via the actuation rod (17) of the piston head (18).

7. Device according to claim 6, wherein the injection unit comprises an angled channel (19d), having an inlet coupled with the bone cement inlet (13c) and an outlet leading to the internal chamber of the elongated body (13) oriented towards the bone cement outlet (23), as well as an opening at the angle of the channel, said opening being adapted so that the piston head (18) passed therethrough to enable the bone cement (100) to be pushed towards said bone cement outlet (23).

8. Device according to any one of claims 6 and 7, wherein the main module (1) comprises, at the distal end (1b) thereof, a screw thread adapted to engage with a complementary screw thread of the injection unit (24) at the inlet (13c) of said unit, to ensure the connection of the injection unit to the main module (1).

9. Device according to any one of claims 3 to 7, wherein the main module (1) comprises, at the proximal end (1a) thereof, a screw thread adapted to engage with a complementary screw thread of the respective caps (4, 8) of the mixing unit (7) and the transfer unit (12).

10. Device according to any one of claims 1 to 9, wherein the distal end (1b) has an opening having a diameter equal to the diameter of a cylinder formed by an internal wall of the main module (1).

11. Method for mixing and injecting a bone cement using a kit-type device comprising a main module (1) with the shape of a hollow cylindrical body having, on the one hand, an internal wall, an open proximal end (1a) and an open distal end (1b) with an opening, the diameter of which is equal to the diameter of the cylinder formed by the internal wall of the hollow cylindrical body, and accessories comprising a removable closing cap (2) and a plurality of functional modules (7, 12, 24), on the other hand, the method comprising:
a) in a mixing phase, connecting one of the functional modules or mixing unit (7) to the proximal end (1a) of the main module while the distal end (1b) of said main module is closed by the closing cap (2), and mixing at least two compounds of the bone cement in the body of the main module (1);
b) in a transfer phase following the mixing phase:
b1) connecting a further functional module or transfer unit (12), distinct from the mixing unit and with no common element with the mixing unit, to the proximal end (1a) of the main module instead of the mixing unit, and
b2) connecting a further functional module or injection unit (24) to the distal end (1b) of the main module instead of the closing cap,
b3) transferring bone cement from the body of the main module to the injection unit; and,
c) in an injection phase following the transfer phase, injecting the bone cement.
